# EUROPÄISCHE PATENTSCHRIFT

(11) **EP 4 389 008 B1**
(45) Veröffentlichungstag und Bekanntmachung des Hinweises auf die Patenterteilung: **30.07.2025**
(21) Anmeldenummer: 23194388.7
(22) Anmeldetag: 30.08.2023
(51) Int. Cl.: A61B 6/00, A61B 6/02, A61B 6/46, A61B 6/50

(54) **VERFAHREN ZUR AUFNAHME EINES GROSSFLÄCHIGEN RÖNTGENBILDES**
METHOD FOR TAKING A LARGE-AREA X-RAY IMAGE
PROCÉDÉ POUR L'ENREGISTREMENT D'UNE IMAGE RADIOLOGIQUE DE GRANDE SURFACE

(30) Priorität: 30.08.2022 DE 102022003163
(43) Veröffentlichungstag der Anmeldung: 26.06.2024
(73) Patentinhaber: Ziehm Imaging GmbH, 90471 Nürnberg (DE)
(72) Erfinder: König, Thomas, 90478 Nürnberg (DE); Wagner, Johannes, 96135 Stegaurach (DE)
(74) Vertreter: Wittmann, Günther

(56) Entgegenhaltungen:
- US-A1- 2012 257 714
- US-A1- 2019 117 180
- US-A1- 2019 175 131
- US-A1- 2021 093 271

## Beschreibung

Die Erfindung betrifft ein Verfahren zur Aufnahme eines großflächigen Röntgenbildes.

Röntgengeräte, beispielsweise mobile oder stationäre C-Bögen, können auf einem 2D-Röntgenbild nur einen begrenzten Bereich eines Patienten abbilden. Dieser Bereich wird vor allem durch die Größe des verwendeten Röntgendetektors, beispielsweise ein Flachdetektor oder ein Bildverstärker, beispielsweise 20x20 cm² oder 30x30 cm², vorgegeben. In den meisten Anwendungsfällen ist es ausreichend eine einfache Röntgenbildaufnahme anzufertigen. Es existieren jedoch auch Anwendungsfälle, die von Abbildungen profitieren, welche über die Größe des verwendeten Detektors hinausgehen. Dazu gehören beispielsweise die Darstellung von Wirbelsäulen und deren krankhafte Veränderungen, beispielsweise Skoliosen (Verkrümmungen entlang der Längsachse) sowie beispielsweise die Verfolgung von Kontrastmittelinjektionen, beispielsweise entlang eines kompletten Beines oder bei Aorten-Interventionen, welche im Folgenden als Bolus-Chase oder Bolus bezeichnet werden.

In den vorangegangenen genannten Anwendungsfällen handelt es sich um interventionelle Anwendungen, bei welchen insbesondere auch der Vergleich vor, während und nach einem operativen Eingriff von Interesse ist. Im Falle von orthopädischen Anwendungen kann dabei im Allgemeinen von einer statischen Szene ausgegangen werden, sodass durch Veränderung des Bildausschnittes, insbesondere mittels einer Verschiebung der Patientenlagerung, beispielsweise einer Patientenliege, Röntgenbilder von verschiedenen Bereichen des Patienten aufgenommen werden können.

Unabhängig davon, wie die Veränderung des Bildausschnitts realisiert wird, handelt es sich zunächst jedoch um einzelne oder vereinzelte Röntgenbilder, welche noch ohne eine Beziehung zueinander existieren und vom Anwender nur einzeln bzw. als Sequenz betrachtet werden können. Insbesondere bei einer kontinuierlichen Änderung der Position der Patientenliege wäre es vorteilhaft, die einzelnen Röntgenbilder zu einem großen Gesamtbild zusammenzuführen.

Eine naheliegende Möglichkeit zur Erzeugung eines großflächigen Röntgenbildes ist aus der DE102019001988 bekannt. Hierbei werden die Einzelaufnahmen einfach aneinandergefügt, beispielsweise mittels eines Algorithmus zur Bildregistrierung, insbesondere sog. "Stitching"-Verfahren zusammengefügt. Bei einem einfachen Zusammenführen von wenigstens zwei Röntgenbildern zu einem großflächigen Röntgenbild kann es jedoch vermehrt zum Auftreten von Artefakten im zu ermittelnden Gesamtbild kommen. Ursachen hierfür können die unvermeidbare Kegelstrahlgeometrie des Röntgengeräts sowie die Verwendung von planaren (ebenen) Detektoren in dieser Geometrie sein. Bei der Verwendung einer Röntgen-Punktquelle wie in herkömmlichen Röntgenröhren sorgt diese nämlich dafür, dass die entsprechenden Bereiche in den z.B. zwei zusammenzufügenden Bildern aus unterschiedlichen Blickwinkeln aufgenommen werden und es somit zu unterschiedlichen Darstellungen von gleichen Objekten kommt. Aus den unterschiedlichen Darstellungen der gleichen Objekte ergibt sich somit, dass die Röntgenbilder mittels eines einfachen Bildregistrierverfahrens nur unzureichend bzw. inkonsistent in Deckung gebracht werden können.

Weiterhin bedingt die Verwendung von planaren Detektoren in Verbindung mit einer Röntgen-Punktquelle, welche divergente Röntgenstrahlen erzeugt, dass Objekte bzw. Patientenbereiche umso stärker verzerrt werden, je weiter sie vom Mittelpunkt des Detektors entfernt sind. In diesem Fall ergibt sich ebenfalls die Situation, dass eine Region, die im einen Bild am Rand des Detektors und im anderen Bild beispielsweise in dessen Mitte liegt, in einem Gesamtbild nicht konsistent dargestellt werden kann.

Weiterhin kann beim Vorliegen von Szenenänderungen bei der Aufnahme, beispielsweise durch das Spritzen von Kontrastmittel bedingt, eine Bildregistrierung nicht mehr erfolgreich durchgeführt werden, da sich durch die zeitliche Veränderung der Szene, beispielsweise durch Ausbreitung des Kontrastmittels, keine hinreichend übereinstimmenden Bildbereiche in der Bildsequenz mehr ergeben.

Das Dokument DE10 2015 204 957 offenbart ein Tomosynthese-System, welches Tiefeninformationen farbkodiert anzeigt.

Das Dokument DE 10 2018 212 389 offenbart ein Tomosynthese-System, welches sich entlang des Patienten bewegt, und wobei ein Kollimator abhängig von der Position des Systems den Kegelstrahlwinkel verändert.

Aus der DE102020209714 ist das Auswählen eines Bereichs von Interesse an einem Touch Display oder GUI bekannt und das darauffolgende Verfahren der Objektlagerung und der Röntgenquelle an genau diese Position. Im Gegensatz zur vorliegenden Erfindung werden Objektlagerung und Röntgenquelle gleichzeitig und in verschiedene Richtungen verfahren.

Aus EP3669942 ist ebenfalls das Auswählen eines Bereichs von Interesse an einem Touch Display oder GUI bekannt und das darauffolgende Verfahren der Objektlagerung und/oder der Röntgenquelle an genau diese Position. Jedoch werden hier mehrere Regionen ausgewählt und mit einem verstellbaren Marker markiert.

Dokument US 2019/175131 A1 offenbart ein Verfahren zur Bereitstellung eines großflächigen Röntgenbildes eines mittels einer Objektlagerung gelagerten Objekts und Bereitstellung weiterer synthetischer Röntgenprojektionen umfassend: Aufnahme einer Mehrzahl von Röntgenprojektionen mittels eines Röntgengeräts, wobei die Mehrzahl von Röntgenprojektionen während einer linearen Bewegung der Objektlagerung und/oder des Röntgengeräts aufgenommen werden, Erstellung wenigstens eines tomosynthetischen Volumens aus der Mehrzahl von Röntgenprojektionen, Erstellung wenigstens einer ersten synthetischen Vorwärtsprojektion aus dem wenigstens einen tomosynthetischen Volumen, wobei die wenigstens eine synthetische Vorwärtsprojektion ein großflächiges Röntgenbild erzeugt und Darstellung des großflächigen Röntgenbildes auf einem geeigneten Anzeigegerät.

Die Aufgabe der Erfindung ist es daher, ein Verfahren zur Verfügung zu stellen, welches es ermöglicht, aus wenigstens zwei Röntgenbildern ein Artefakt- und verzerrungsfreies großflächiges Röntgenbild zur Verfügung zu stellen, welches auf geeigneten Anzeigegeräten, wie Bildschirmen, Displays und/oder Computern dargestellt werden kann. Vorzugsweise können diese Anzeigegeräte eine Touch-, Front-End oder GUI-Funktion beinhalten. Die nachfolgend genannte Objektlagerung kann einer Patientenliege oder jeder anderen geeigneten Lageeinrichtung für Patienten und/oder Gegenständen entsprechen.

Die Aufgabe der Erfindung wird erfindungsgemäß durch die Merkmale des unabhängigen Anspruchs gelöst. Vorteilhafte Ausbildungen werden durch die abhängigen Patentansprüche angegeben.

Vorzugsweise wird die Erfindung mit dem Zweck, ein großflächiges Röntgenbilde zur Verfügung zu stellen, softwaremäßig umgesetzt. Eine weitgehend softwaremäßige Umsetzung des Verfahrens hat den Vorteil, dass auch bereits bisher für diesen Zweck verwendete Verfahren für Bildaufnahmesysteme auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung einer Röntgenvorrichting, beispielsweise einer C-Bogen-Röntgenvorrichtung, ladbar ist, mit Programmabschnitten, um alle Schritte des erfindungsgemäßen Verfahrens auszuführen, wenn das Computerprogramm in der Steuerungseinrichtung ausgeführt wird. Ein solches Computerprogrammprodukt kann neben dem Computerprogramm gegebenenfalls zusätzliche Bestandteile wie beispielsweise eine Dokumentation und/oder zusätzliche Komponenten, auch Hardware-Komponenten zur Nutzung der Software, umfassen.

Zum Transport zur Steuerungseinrichtung und/oder zur Speicherung an oder in der Steuerungseinrichtung kann ein computerlesbares Medium, beispielsweise ein Memorystick, eine Festplatte oder ein sonstiger transportabler oder fest eingebauter Datenträger dienen, auf welchem die von einer Rechnereinheit der Steuerungseinrichtung einlesbaren und ausführbaren Programmabschnitte des Computerprogramms gespeichert sind. Dem Transport kann auch eine Verbindung zu einem an einem Netzwerk angeschlossenen Krankenhausinformationssystem, zu einem Radiologieinformationssystem oder zu einem globalen Netz dienen, in welchen Systemen die von einer Rechnereinheit der Steuerungseinrichtung einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Zur Lösung des Problems wird ein Verfahren zur Aufnahme eines großflächigen Röntgenbildes, insbesondere eines Patienten, auf einer Objektlagerung mittels eines Röntgengerätes, welches Röntgenstrahlen in einer Fächerstrahlgeometrie erzeugt, verwendet, aufweisend die nachfolgenden Schritte:
a. Aufnahme eines ersten Röntgenbildes unter Verwendung einer ersten Aufnahmegeometrie;
b. Aufnahme wenigstens einer zweiten Röntgengeometrie unter Verwendung einer zweiten Aufnahmegeometrie, wobei das erste Röntgenbild teilweise mit dem zweiten Röntgenbild überlappt;
c. Rekonstruktion eines tomosynthetischen Volumens aus den Aufnahmen der wenigstens zwei Röntgenbilder;
d. Erstellung von synthetischen Vorwärtsprojektionen aus dem tomosynthetischen Volumen, wobei die Gesamtheit der synthetischen Vorwärtsprojektionen ein großflächiges Röntgenbild erzeugt;
e. Darstellung des großflächigen Röntgenbildes auf einem Anzeigegerät;
f. Markierung eines Bereichs von Interesse auf einem geeigneten Anzeigegerät,
g. Erstellung einer synthetischen Vorwärtsprojektion des Bereichs von Interesse als Vorschaubild, basierend auf einer Auswertung der relativen Lage des Röntgengerätes und der Objektlagerung, sodass das Vorschaubild in einer Aufnahmegeometrie angefertigt wird, die der gegenwärtigen Orientierung des Röntgengeräts im Raum entspricht.

Dem erfindungsgemäßen Verfahren liegt eine erste Aufnahme eines Röntgenbildes zugrunden, welche unter Verwendung einer ersten Aufnahmegeometrie angefertigt wurde. Bei der Aufnahmegeometrie handelt es sich insbesondere um die Position (Stellung) des Röntgengeräts zu der Objektlagerung, wobei die Aufnahmegeometrie beispielsweise durch eine Verdrehung oder durch eine translatorische Verstellung der Lage des Röntgengeräts zur Objektlagerung, beispielsweise durch ein Heben oder Senken des Röntgengenerators und/oder des Röntgendetektors sowie beispielsweise ein translatorisches Verstellen des Röntgengenerators parallel und/oder senkrecht in einer Ebene zu verstellen ist. Dem erfindungsgemäßen Verfahren liegt die Aufnahme eines zweiten Röntgenbildes zugrunde, wobei die Aufnahmegeometrie des ersten Röntgenbildes und die Aufnahmegeometrie des zweiten Röntgenbildes voneinander unterschiedlich sind. Vorzugsweise unterscheidet sich die Aufnahmegeometrie des zweiten Röntgenbildes von der Aufnahmegeometrie des ersten Röntgenbildes durch einen translatorischen Versatz entlang der Richtung der Objektlagerung. Nach dem erfindungsgemäßen Verfahren ist es unerheblich, ob die Änderung der Aufnahmegeometrie aus einer Änderung der Lage des Röntgengeräts oder aus der Änderung der Lage der Objektlagerung (relative Lageänderung) resultiert.

Anschließend wird aus dem ersten und dem zweiten Röntgenbild ein tomosynthetisches 3D-Volumen rekonstruiert, wobei das tomosynthetische 3D-Volumen im Gegensatz zur 3D-Computertomographie eine unvollständige Winkelabdeckung und damit eine beschränkte Tiefenauflösung aufweist. Das tomosynthetische Volumen kann dabei mittels bekannter Verfahren, insbesondere mit Hilfe einer gefilterten oder ungefilterten Rückprojektion, eines iterativen oder eines algebraischen Rekonstruktionsverfahrens, oder auch durch Anwendung eines zu diesem Zwecke trainierten Maschinenlernverfahrens erzeugt werden. Optional ist vorgesehen, hierbei eine Kalibrierung des C-Bogens, der Objektlagerung und/oder insbesondere der relativen Positionierung bzw. Ausrichtung beider zueinander, zu verwenden. Alternativ kann eine Rekonstruktion auch ohne eine solche Kalibrierung durchgeführt werden.

Nach der Rekonstruktion eines tomosynthetischen 3D-Volumens erfolgt die Erstellung von synthetischen Vorwärtsprojektionen vorzugsweise in Parallel- bzw. Fächerstrahlgeometrie aus dem tomosynthetischen Volumen, wobei die Gesamtheit aller synthetischen Vorwärtsprojektionen zusammengeführt ein großflächiges Röntgenbild ergeben. Das so erzeugte großflächige Röntgenbild wird im Anschluss auf einem Anzeigegerät dargestellt. Es ist weiterhin vorgesehen, die Vorwärtsprojektion alternativ als Kegelstrahlgeometrie durchzuführen.

In alternativen Ausgestaltungen umfasst das erfindungsgemäße Verfahren eine Zoom-Funktionalität insbesondere für das großflächige Röntgenbild, beispielsweise für eine Vergrößerung oder Verkleinerung von Bereichen des großflächigen Röntgenbildes, wobei ein Bereich nicht zwingend einer Aufnahme entsprechen muss, sondern ein Bereich auch aus mehreren Röntgenaufnahmen bestehen kann.

Nachfolgend sieht es das erfindungsgemäße Verfahren vor, dass basierend auf einer Auswertung der relativen Lage des Röntgengeräts und der Objektlagerung eine synthetische Vorwärtsprojektion als Vorschau bzw. Vorschaubild auf einem Anzeigegerät dargestellt wird. Bei der Berechnung des Vorschaubildes wird hierbei die relative Lage des Röntgengeräts zur Objektlagerung derart ausgewertet, dass das Vorschaubild einem in dieser Lage aufgenommenen Röntgenbild zumindest näherungsweise entspricht, insbesondere der dabei vorliegenden Aufnahmegeometrie. Erfindungsgemäß ist es daher vorgesehen, diese synthetische Vorwärtsprojektion vorzugsweise in einer Kegelstrahlgeometrie zu erstellen, welche den Abstand von Röntgenfokus und Röntgendetektor berücksichtigt. Dies ist vorteilhaft, um das Vorschaubild in einer Art und Weise zu generieren, die einem tatsächlich aufgenommenen Röntgenbild möglichst nahekommt. Eine bevorzugte Ausgestaltungsform des erfindungsgemäßen Verfahrens sieht daher vor, bei der Verstellung der Orientierung des Röntgengeräts im Raum durch Benutzer automatisch das Vorschaubild zu aktualisieren, sodass dieses weiterhin die aktuelle Orientierung des Röntgengeräts widerspiegelt.

Zur Reduktion der Strahlendosis für Patienten sieht eine bevorzugte Ausgestaltungsform des erfindungsgemäßen Verfahrens darüber hinaus vor, ein Vorschaubild zu generieren, welches nicht der aktuellen relativen Lage des Röntgengeräts und der Objektlagerung entspricht, sondern einer für den gewählten medizinischen Arbeitsablauf besonders vorteilhaften. Hierbei ist es vorgesehen, dass Anwender einen Bereich von Interesse entsprechend der Anfordernisse und auf Basis des großflächigen Röntgenbildes auswählen, und in der Folge eine automatische, vorzugsweise motorische Verstellung der Objektlagerung und/oder des Röntgengeräts derart erfolgt, dass sich nach der Verstellung die gewünschte Aufnahmegeometrie ergibt.

Eine weitere bevorzugte Ausgestaltungsform sieht vor, dass das Errechnen einer Bewegung des Röntgengeräts und/oder der Objektlagerung aus dem empfangenen Bereich von Interesse unter der Maßgabe erfolgt, dass sich nach Ausführung der Bewegung eine Projektionsgeometrie relativ zum Objekt ergibt, welche nachfolgend das Anfertigen einer Röntgenprojektion erlaubt, die zumindest näherungsweise der zweiten synthetischen Vorwärtsprojektion entspricht. Anschließend wird die errechnete Bewegung dahingehend ausgewertet, ob sie durch die kinematischen Randbedingungen des Röntgengeräts und/oder der Objektlagerung durchführbar ist. Schließlich wird die Bewegung nur durchgeführt, falls diese durchführbar ist. Andernfalls ist die Ausgabe eines Hinweises in visueller und/oder akustischer Form vorgesehen, falls die Bewegung nicht durchführbar ist.

In alternativen Ausgestaltungen werden die obigen Schritte jedoch nur dann durchgeführt, wenn ein Sicherheitskriterium erfüllt ist, insbesondere, wenn aufgrund der bestimmten relativen Lage zumindest näherungsweise sichergestellt ist, dass alle für diese Vorwärtsprojektion benötigen Frequenzen in der Fourier-Repräsentation des tomosynthetischen Volumens enthalten sind und damit die Vorwärtsprojektion weitgehend Artefakt frei erstellt werden kann. Ist dies nicht der Fall, kann eine Meldung auf dem Anzeigegerät ausgegeben werden, die darauf hinweist, dass eine gewählte Aufnahmegeometrie für die Erstellung eines Vorschaubildes nicht geeignet ist.

In alternativen Ausgestaltungen umfasst das erfindungsgemäße Verfahren Funktionen zur Messung von Längen und/oder Winkeln auf den synthetisch generierten Vorwärtsprojektionen, insbesondere auf dem großflächigen Röntgenbild.

In alternativen Ausgestaltungen kann das erfindungsgemäße Verfahren eine Sequenz der aufgenommenen Röntgenbilder auf einem Anzeigegerät anzeigen, beispielsweise in einer Form einer Filmsequenz, vorzugsweise kombiniert mit einer Hervorhebung, insbesondere einer Visualisierung eines Teils der Sequenz, welche der aktuellen Position des Röntgengeräts zur Objektlagerung entspricht.

In alternativen Ausgestaltungen des erfindungsgemäßen Verfahrens ist es vorgesehen, dass basierend auf der aktuellen Geschwindigkeit der Positionsänderung, beispielsweise der Bewegung der Patientenliege oder der Bewegung des Röntgengeräts oder die relative Bewegung zwischen Röntgengerät und Patientenliege, es zur Anpassung der zeitlichen Pulsrate und Pulsdauer des Röntgengeräts führen kann, vorzugsweise um eine für die Tomosynthese geeignete Abtastrate zu ermitteln und Bewegungsunschärfe in den aufgenommenen Röntgenbildern zu verhindern oder abzuschwächen.

Das erfindungsgemäße Verfahren findet ebenfalls Anwendung im Falle einer Änderung einer Szene (Szenenänderung) während der Aufnahme der Röntgenbilder für das großflächige Röntgenbild. Insbesondere können diese Szenenänderungen bedingt durch die Injektion von Kontrastmittel oder Kontrastmitteln erzeugt werden. Dabei können sichtbare Artefakte in den aufgenommenen Röntgenbildern entstehen, da sich aufgrund von zeitlichen Änderungen, insbesondere einer zeitlich veränderlichen Kontrastmitteldynamik, Inkonsistenzen zwischen den einzelnen Röntgenbildern ergeben. Diese ergeben sich daraus, dass die Szenenänderungen, beispielsweise verursacht aufgrund des ein- und ausfließenden Kontrastmittels, zu unterschiedlichen Zeitpunkten in den Röntgenbildern wiedergegeben sind. Vor Anfertigung des großflächigen Röntgenbildes werden dabei die jeweiligen Szenenänderungen auf den aufgenommenen Röntgenbildern identifiziert, also diejenigen Bildbereiche, die ein dynamisches Geschehen aufweisen, beispielsweise im Falle von injizierten Kontrastmitteln die Blutgefäße, wobei die Blutgefäße wenigstens teilweise mit Kontrastmittel gefüllt sind. Erfindungsgemäß gilt die Relativbewegung von Röntgengerät und Objektlagerung dabei nicht als eine Szenenänderung, da diese im Rahmen der Tomosynthese entsprechend behandelt wird. Relevante Szenenänderung können dabei in vorteilhafter Weise mittels Bildverarbeitungsverfahren identifiziert werden. Hierfür können klassische Verfahren oder aber auch zuvor trainierte Maschinenlernverfahren zum Einsatz kommen, die jeweils an den Anwendungsfall angepasst sind, beispielsweise an den Fluss eines Kontrastmittels. Vorzugsweise wird hierbei aus einer Ausgangsröntgenprojektion jeweils ein Satz mit mindestens einer abgeleiteten Projektion, vorzugsweise jedoch mehrerer solcher erstellt. Die abgeleiteten Projektionen entsprechen dabei bestimmten Klassen von Bildinhalten, insbesondere den Bildinhalten ohne Szenenänderungen sowie einer oder mehrerer Klassen von Szenenänderungen, beispielsweise den mit Kontrastmittel gefüllten Blutgefäßen. In diesen alternativen Ausgestaltungen des erfindungsgemäßen Verfahrens werden die identifizierten Szenenänderungen dann wie folgt berücksichtigt:
Basierend auf den erstellten Sätzen aus der mindestens einen abgeleitete Projektion wird mindestens eine tomosynthetische Rekonstruktion erstellt, und zwar basierend auf den zu einer bestimmten Klasse gehörigen, abgeleiteten Projektionen. In einer möglichen Ausgestaltung des erfindungsgemäßen Verfahrens wird eine tomosynthetische Rekonstruktion erstellt ausschließlich unter Berücksichtigung der Klasse, die denjenigen Bildbereichen entspricht, die keine Szenenänderung erfahren haben, beispielsweise die umgebende Anatomie eines Blutgefäßes, insbesondere Knochen und/oder Weichteile. Diese tomosynthetische Rekonstruktion stellt die Grundlage für die Erstellung eines großflächigen Röntgenbildes mittels Vorwärtsprojektion dar, welches als Hintergrund interpretiert werden kann.

In alternativen Ausgestaltungen des erfindungsgemäßen Verfahrens kann basierend auf weiteren abgeleiteten Projektionen eine tomosynthetische Rekonstruktion erstellt werden, welche nur eine bestimmte Klasse von Szenenänderungen beinhaltet, beispielsweise Blutgefäße mit Kontrastmittel. Für die vorangegangenen beschriebenen Verfahrensschritte ist es vorteilhaft, über ein Vorwissen zu verfügen, ob Szenenänderungen vorliegen könnten und diese rechnerisch zu verwerten sind. Dies kann beispielsweise durch das ausgewählte Organprogramm erfolgen, sodass nur im entsprechenden Fall das erfindungsgemäße Verfahren nach Szenenänderungen suchen wird. Beispielsweise würde bei der Anfertigung von Wirbelsäulen-Aufnahmen nicht nach Szenenänderungen gesucht werden, während bei der Aufnahme eines Bolus, beispielsweise bei injiziertem Kontrastmittel im Gefäßbereich eines Patienten, nach einer Szenenänderung gesucht wird.

Die abgeleiteten Projektionen können über verschiedene Verfahren insbesondere mittels bildverarbeitungstechnischen Methoden berechnet werden. Im Falle von Kontrastmittelhaltigen Regionen können diese beispielsweise über eine Helligkeitsänderung detektiert werden. Alternativ können dunkle Bildregionen, welche scharf begrenzt und in Bewegungsrichtung orientiert sind, als Kontrastmittelhaltige Blutgefäße identifiziert werden, beispielsweise mittels eines Strukturtensors. Ganz allgemein können auch Verfahren des maschinellen Lernens, beispielsweise Deep Learning, insbesondere Convolutional Neural Networks trainiert werden, um beispielsweise Bereiche mit einer Szenenänderung über die Bildsequenz hinweg zu identifizieren. Eine Koordinatentransformation zwischen aufeinanderfolgenden Bildern kann dabei insbesondere über Auswertung von Positions-, Winkel- und/oder Geschwindigkeitsencodern erfolgen, beispielsweise um Helligkeitsänderungen zu identifizieren.

In diesen Ausgestaltungen kann die synthetische Vorwärtsprojektion dann insbesondere in Parallelstrahl- oder Fächerstrahlgeometrie für alle tomosynthetischen Rekonstruktionen erzeugt werden. Das großflächige 2D-Röntgenbild kann somit mit und ohne Szenenänderungen, beispielsweise kontrastmittelhaltige Blutgefäße, dargestellt werden. Insbesondere ist es möglich, zwischen den verfügbaren Darstellungen zu wechseln, vorzugsweise den kontrastmittelhaltigen Gefäßbaum oder den Patientenhintergrund ein- und auszublenden.

Dem erfindungsgemäßen Verfahren liegt eine erste Aufnahme eines Röntgenbildes zugrunde, welche unter Verwendung einer ersten Aufnahmegeometrie angefertigt wurde. Bei der Aufnahmegeometrie handelt es sich insbesondere um die Position (Stellung) des Röntgengeräts zu der Objektlagerung, wobei die Aufnahmegeometrie beispielsweise durch eine Verdrehung oder durch eine translatorische Verstellung der Lage des Röntgengeräts zur Objektlagerung, beispielsweise durch ein Heben oder Senken des Röntgengenerators und/oder des Röntgendetektors sowie beispielsweise ein translatorisches Verstellen des Röntgengenerators parallel und/oder senkrecht in einer Ebene zu verstellen ist. Dem erfindungsgemäßen Verfahren liegt die Aufnahme eines zweiten Röntgenbildes zugrunde, wobei die Aufnahmegeometrie des ersten Röntgenbildes und die Aufnahmegeometrie des zweiten Röntgenbildes voneinander unterschiedlich sind. Vorzugsweise unterscheidet sich die Aufnahmegeometrie des zweiten Röntgenbildes von der Aufnahmegeometrie des ersten Röntgenbildes durch einen translatorischen Versatz entlang der Richtung der Objektlagerung. Nach dem erfindungsgemäßen Verfahren ist es unerheblich, ob die Änderung der Aufnahmegeometrie aus einer Änderung der Lage des Röntgengeräts oder aus der Änderung der Lage der Objektlagerung (relative Lageänderung) resultiert.

Anschließend wird aus dem ersten und dem zweiten Röntgenbild ein tomosynthetisches 3D-Volumen rekonstruiert, wobei das tomosynthetische 3D-Volumen verfahrensbedingt/technisch bedingt eine beschränkte Tiefenauflösung besitzt. Das tomosynthetische Volumen kann dabei mittels bekannter Verfahren, insbesondere mit Hilfe einer gefilterten Rückprojektion, eines iterativen oder eines algebraischen Rekonstruktionsverfahrens, oder auch durch Anwendung eines zu diesem Zwecke trainierten Maschinenlernverfahrens erzeugt werden. Optional ist vorgesehen, hierbei eine Kalibrierung des C-Bogens, der Patientenliege und insbesondere der relativen Positionierung bzw. Ausrichtung beider zueinander, zu verwenden. Alternativ kann eine Rekonstruktion auch ohne eine solche Kalibrierung durchgeführt werden.

Nach der Rekonstruktion eines tomosynthetischen 3D-Volumens erfolgt die Erstellung von synthetischen Vorwärtsprojektionen vorzugsweise in Parallel- oder Fächerstrahlgeometrie aus dem tomosynthetischen Volumen, wobei die Gesamtheit aller synthetischen Vorwärtsprojektionen zusammengeführt ein großflächiges Röntgenbild ergibt. Das so erzeugte großflächige Röntgenbild wird im Anschluss auf einem Anzeigegerät dargestellt. Es ist weiterhin in alternativen Ausgestaltungen vorgesehen, die Vorwärtsprojektion alternativ in Parallel-, Fächer- oder Kegelstrahlgeometrie durchzuführen.

Die Erfindung wird anhand der nachfolgenden Figurenbeschreibungen näher erläutert.
Figur 1 zeigt eine erfindungsgemäße Vorrichtung in Ausgestaltung eines mobilen C-Bogens.
Figur 2 stellt schematisch das Verfahren der Patientenliege dar.
Figur 3 offenbart die synthetische Vorwärtsprojektion in Parallel- und Kegelstrahlgeometrie.

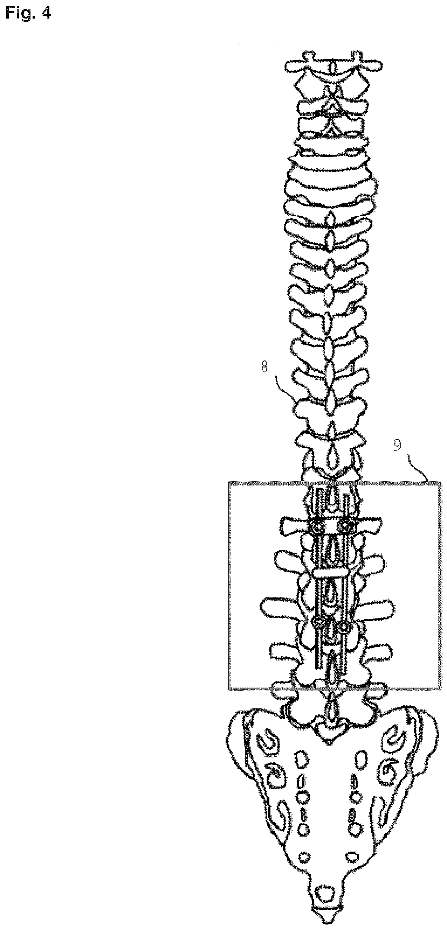

Figur 4 stellt schematisch das zu durchleuchtende Objekt dar inkl. Eines Bereichs von Interesse.

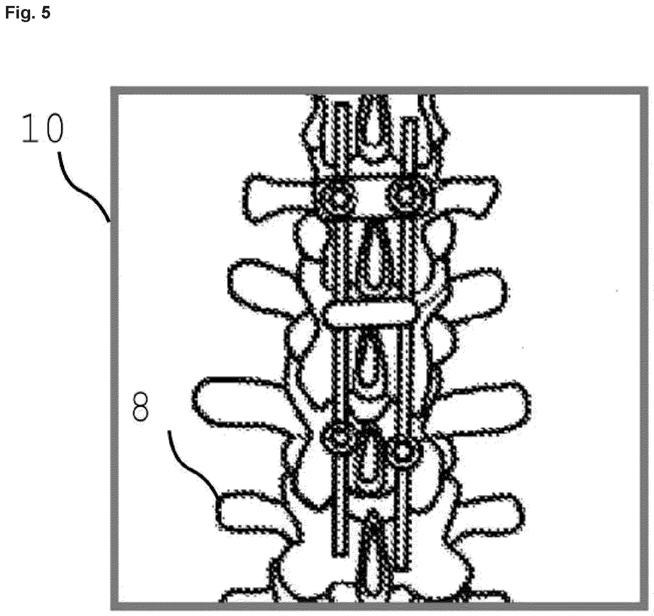

Figur 5 zeigt das zu dem Bereich von Interesse generierte Vorschaubild.

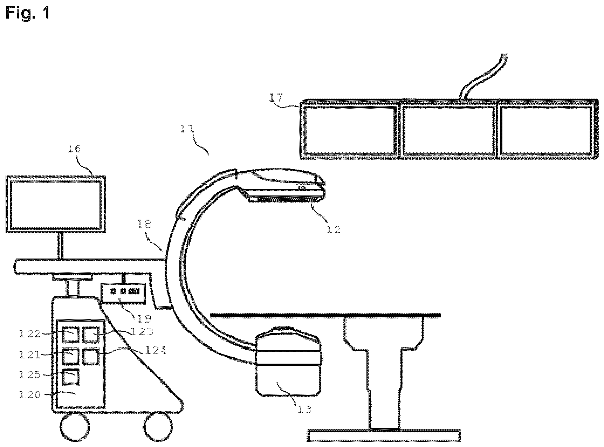

In Fig. 1 ist eine erfindungsgemäße Vorrichtung in Ausgestaltung eines mobilen C-Bogens 11 schematisch dargestellt, welcher für die Umsetzung des erfindungsgemäßen Verfahrens vorgesehen ist.

Der C-Bogen 11 trägt an seinem einen Ende einen Röntgengenerator 13, und an seinem anderen Ende und dem Röntgengenerator 13 gegenüberliegend einen Röntgenbilddetektor 12, beispielsweise kann es sich hierbei um einen Flachdetektor oder einen Bildverstärker handeln. Der C-Bogen 11 ist in mehreren Achsen im Raum motorisch gesteuert verstellbar, wobei die Achsen Sensoren zur Erfassung des Maßes der Verstellung aufweisen.

Ferner beinhaltet die Vorrichtung eine Bildverarbeitungseinheit 121, eine Speichereinheit 122, einer Steuerungseinheit 123, sowie eine Netzwerkschnittstelle 124. Mittels der Netzwerkschnittstelle 124 können Daten, beispielsweise Bilddatensätze und Resultate des erfindungsgemäßen Verfahrens, in einem Netzwerk verteilt bzw. zur Verfügung gestellt werden.

Die Bildverarbeitungseinheit 121 umfasst eine Steuerungseinheit 122, auf dieser können die für das erfindungsgemäße Verfahren verwendeten zwei- oder dreidimensionalen Bilddatensätze gespeichert bzw. geladen werden. Diese Bilddatensätze können entweder von einem Server geladen oder mittels des C-Bogen 11 vor oder während einer Intervention aufgenommen werden. Ferner umfasst die Speichereinheit Instruktionen, die für die Ausführung des erfindungsgemäßen Verfahrens mittels einer Recheneinheit verwendet werden.

Ferner kann die Vorrichtung eine GUI beinhalten, mit einer Bildausgabeeinheit (16, 17) und einer Eingabeeinheit 19, mit welchen für die Bildverarbeitungseinheit 121 entsprechende Einstellungen in entsprechenden Organprogrammen vorgenommen werden können.

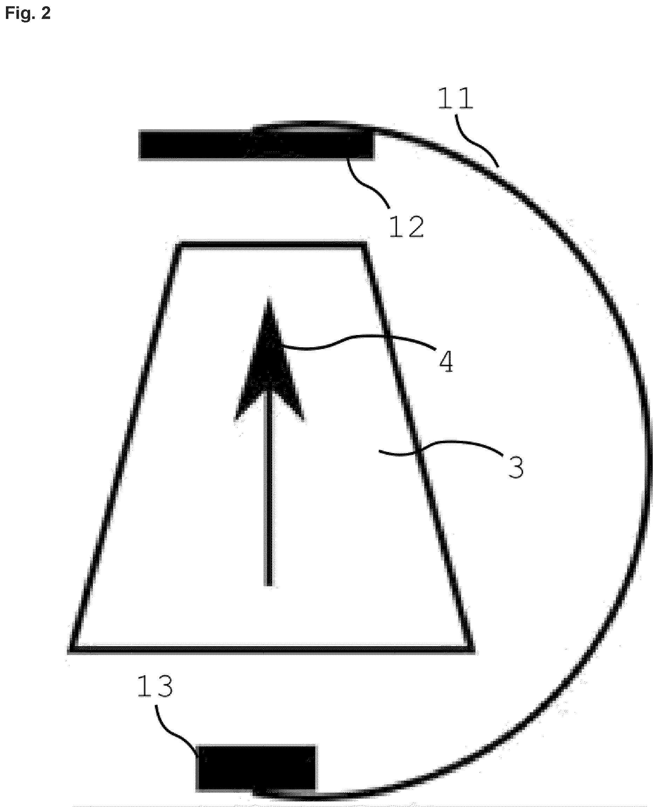

In Fig. 2 ist eine erfindungsgemäße Vorrichtung in Ausgestaltung eines mobilen C-Bogens 11 schematisch dargestellt, gemeinsam mit einer in mehreren Bewegungsrichtungen 4 verstellbaren/verschiebbaren/verfahrbaren Objektlagerung 3. Die Objektlagerung 3 kann durch den C-Bogen 11 vor und zurück bewegt werden, wobei deren Achsen Sensoren zur Erfassung des Maßes der Verstellung aufweisen.

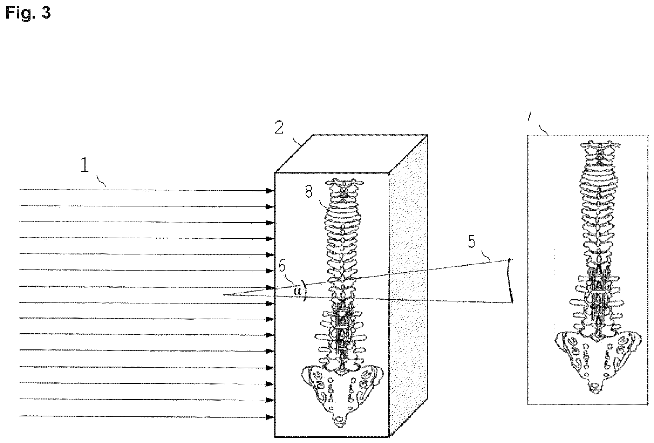

In Fig. 3 ist die synthetische Vorwärtsprojektion als großflächiges Röntgenbild zu sehen. Hierbei werden nach Erstellung des tomosynthetischen Volumens 2 eine oder mehrere synthetische Vorwärtsprojektion aus diesem erstellt, indem beispielsweise softwaremäßig bzw. in einem Computerprogramm Parallelstrahlen 1 durch das tomosynthetische Volumen 2 geschickt werden, um das großflächige Röntgenbild bzw. Panoramabild 7 zur Verfügung zu stellen. Auf diesem auf dem Bildschirm 16 dargestellten Panoramabild 7 wird nun der Bereich von Interesse 9 ausgewählt und für diesen das Vorschaubild 10 vorzugsweise in Kegelstrahlgeometrie mit dem Öffnungswinkel der Zentralprojektion 6 berechnet, welches daraufhin auf einem geeigneten Anzeigegerät 16 dargestellt werden kann und wobei das Vorschaubild 10 in einer solchen Projektionsgeometrie erstellt wird, die der gegenwärtigen Orientierung des Röntgengeräts im Raum entspricht. Erfindungsgemäß kann dieses Anzeigegerät 16 ein Touch- oder GUI-fähiger Bildschirm sein. Jetzt können mittels eines Eingabebefehls die Objektlagerung und/oder der C-Bogen 11 direkt zu diesem Bereich von Interesse 9 verfahren werden und an diesem ein tatsächliches Röntgenbild aufgenommen werden, welches dem zuvor erstellten Vorschaubild nahekommt, und welches erfindungsgemäß dargestellt werden kann.

Bild 4 zeigt das großflächige Röntgenbild 7 und den ausgewählten Bereich von Interesse 9.

Bild 5 zeigt das aus dem Bereich von Interesse 9 erfindungsgemäß generierte und dargestellte Vorschaubild 10 des zu durchleuchtenden Objektes 8.

### Bezugszeichenliste

- 1: Parallelprojektion bzw. Projektion in Parallelstrahlgeometrie
- 2: Tomosynthetisches Volumen
- 3: Objektlagerung
- 4: Bewegungsrichtung
- 11: C-Bogen
- 12: Röntgenbilddetektor
- 13: Röntgengenerator
- 16, 17: Bildausgabeeinheit
- 19: Eingabeeinheit
- 121: Bildverarbeitungseinheit
- 122: Speichereinheit
- 123: Recheneinheit
- 124: Netzwerkschnittstelle
- 5: Zentralprojektion bzw. Projektion in Kegelstrahlgeometrie
- 6: Öffnungswinkel der Zentralprojektion
- 7: Großflächiges Röntgenbild bzw. Panoramabild
- 8: Objekt
- 9: Bereich von Interesse
- 10: Vorschaubild

## Patentansprüche

1. Verfahren zur Bereitstellung eines großflächigen Röntgenbildes eines mittels einer Objektlagerung gelagerten Objekts und Bereitstellung weiterer synthetischer Röntgenprojektionen, umfassend:
• Aufnahme einer Mehrzahl von Röntgenprojektionen mittels eines Röntgengeräts, wobei die Mehrzahl von Röntgenprojektionen während einer linearen Bewegung der Objektlagerung und/oder des Röntgengeräts aufgenommen werden;
• Erstellung wenigstens eines tomosynthetischen Volumens aus der Mehrzahl von Röntgenprojektionen;
• Erstellung wenigstens einer ersten synthetischen Vorwärtsprojektion aus dem wenigstens einen tomosynthetischen Volumen, wobei die wenigstens eine synthetische Vorwärtsprojektion ein großflächiges Röntgenbild erzeugt;
• Darstellung des großflächigen Röntgenbildes auf einem geeigneten Anzeigegerät;
**dadurch gekennzeichnet, dass** das Verfahren weiter umfasst:
• Auswählen und/oder markieren eines Bereichs von Interesse innerhalb des großflächigen Röntgenbildes;
• Erstellen wenigstens einer zweiten synthetischen Vorwärtsprojektion umfassend den Bereich von Interesse;
• Darstellung der wenigstens einen zweiten synthetischen Vorwärtsprojektion auf einem geeigneten Anzeigegerät;
wobei die wenigstens eine zweite synthetische Vorwärtsprojektion in einer solchen Projektionsgeometrie erstellt wird, die der gegenwärtigen Orientierung des Röntgengeräts im Raum entspricht.

2. Verfahren nach Anspruch 1, **dadurch gekennzeichnet, dass** der linearen Bewegung der Objektlagerung und/oder des Röntgengeräts eine weitere Bewegung überlagert ist.

3. Verfahren nach einem der vorstehenden Ansprüche, **dadurch gekennzeichnet, dass** die Bewegung der Objektlagerung und/oder des Röntgengeräts durch Ermitteln von Positions- und/oder Orientierungsinformationen erfasst wird und die Rekonstruktion des wenigstens einen tomosynthetischen Volumens unter deren Berücksichtigung erfolgt.

4. Verfahren nach einem der vorstehenden Ansprüche, **dadurch gekennzeichnet, dass** die Erstellung des wenigstens einen tomosynthetischen Volumens mittels einer zuvor erfolgten Kalibrierung der Aufnahmegeometrie erfolgt.

5. Verfahren nach einem der vorstehenden Ansprüche, **dadurch gekennzeichnet, dass** die Erstellung der wenigstens einen zweiten synthetischen Vorwärtsprojektion in einer Kegelstrahlgeometrie erfolgt.

6. Verfahren nach einem der vorstehenden Ansprüche, umfassend:
• Errechnen einer Bewegung des Röntgengeräts und/oder der Objektlagerung aus dem empfangenen Bereich von Interesse unter der Maßgabe, dass sich nach Ausführung der Bewegung eine Projektionsgeometrie relativ zum Objekt ergibt, welche nachfolgend das Anfertigen einer Röntgenprojektion erlaubt, die zumindest näherungsweise der zweiten synthetischen Vorwärtsprojektion entspricht;
• Auswertung, ob die errechnete Bewegung durch die kinematischen Randbedingungen des Röntgengeräts und/oder der Objektlagerung durchführbar ist; und
• Durchführen der Bewegung, falls diese durchführbar ist, oder Nicht-Durchführen der Bewegung und/oder Ausgabe eines Hinweises, falls diese nicht durchführbar ist.

7. Verfahren nach einem der vorstehenden Ansprüche, **dadurch gekennzeichnet, dass** eine Zoom-Funktion für das großflächige Röntgenbild und/oder der zweiten synthetischen Röntgenprojektion bereitgestellt wird.

8. Verfahren nach einem der vorstehenden Ansprüche, **dadurch gekennzeichnet, dass** Funktionen zur Messung von Längen und/oder Winkeln auf der Darstellung des großflächigen Röntgenbildes und/oder der zweiten synthetischen Vorwärtsprojektion bereitgestellt werden.

9. Verfahren nach einem der vorstehenden Ansprüche, **dadurch gekennzeichnet, dass** der durch die zweite synthetische Vorwärtsprojektion dargestellte Objektbereich zumindest näherungsweise in der Darstellung des großflächigen Röntgenbilds markiert wird.

10. Verfahren nach einem der vorstehenden Ansprüche, **dadurch gekennzeichnet, dass** durch Objektveränderungen verursachte Szenenänderungen berücksichtigt werden.

11. Verfahren nach einem der vorstehenden Ansprüche, **dadurch gekennzeichnet, dass** die Erstellung eines tomosynthetischen Volumens aus abgeleiteten Projektionen erfolgt, welche wenigstens eine bestimmte Klasse von Szenenänderung beinhalten, und/oder wenigstens eine bestimmte Klasse von Szenenänderung ausschließen.

12. Verfahren nach einem der Ansprüche 10 oder 11, **dadurch gekennzeichnet, dass** die Szenenänderungen durch eine zeitliche Dynamik einer Kontrastmittelinjektion verursacht werden.

13. Verfahren nach den Ansprüchen 11 und 12, **dadurch gekennzeichnet, dass** wenigstens zwei tomosynthetische Volumina erstellt werden, wobei eines dieser Volumina dem Zustand des Objekts ohne Kontrastmittel entspricht, und ein anderes nur das Kontrastmittel enthält.

14. Verfahren nach einem der vorstehenden Ansprüche, **dadurch gekennzeichnet, dass** die Mehrzahl an Röntgenprojektionen und/oder daraus abgeleiteter Projektionen innerhalb einer zeitlichen Sequenz dargestellt werden.

15. Verfahren nach einem der vorstehenden Ansprüche, **dadurch gekennzeichnet, dass** die Position der aktuell dargestellten Röntgenprojektion und/oder abgeleiteten Projektion in der Darstellung des großflächigen Röntgenbildes markiert wird.

16. Verfahren nach einem der vorstehenden Ansprüche, **dadurch gekennzeichnet, dass** die zeitliche Rate für die Aufnahme der Mehrzahl an Röntgenprojektionen und/oder die Pulsdauer des Röntgengerätes an die Bewegung angepasst wird, insbesondere an deren Geschwindigkeit und/oder Winkelgeschwindigkeit.

17. Röntgenvorrichtung, insbesondere C-Bogen zur Bereitstellung eines großflächigen Röntgenbildes eines mittels einer Objektlagerung gelagerten Objekts und Bereitstellung weiterer synthetischer Röntgenprojektionen, mit:
- einem Computerprogrammprodukt, das in eine Speichereinrichtung der Röntgenvorrichtung ladbar ist;
wobei das Computerprogrammprodukt Programmabschnitte aufweist, um folgende Verfahrensschritte auszuführen:
• Aufnahme einer Mehrzahl von Röntgenprojektionen mittels eines Röntgengeräts, wobei die Mehrzahl von Röntgenprojektionen während einer linearen Bewegung der Objektlagerung und/oder des Röntgengeräts aufgenommen werden;
• Erstellung wenigstens eines tomosynthetischen Volumens aus der Mehrzahl von Röntgenprojektionen;
• Erstellung wenigstens einer ersten synthetischen Vorwärtsprojektionen aus dem wenigstens einen tomosynthetischen Volumen, wobei die wenigstens eine synthetische Vorwärtsprojektion ein großflächiges Röntgenbild erzeugt;
• Darstellung des großflächigen Röntgenbildes auf einem geeigneten Anzeigegerät;
**dadurch gekennzeichnet, dass** dias Computerprogrammprodukt Programmabschnitte aufweist, um folgende weitere Verfahrensschritte auszuführen:
• Auswählen und/oder markieren eines Bereichs von Interesse innerhalb des großflächigen Röntgenbildes;
• Erstellen wenigstens einer zweiten synthetischen Vorwärtsprojektion umfassend den Bereich von Interesse;
• Darstellung der wenigstens einen zweiten synthetischen Vorwärtsprojektion auf einem geeigneten Anzeigegerät;
wobei die wenigstens eine zweite synthetische Vorwärtsprojektion in einer solchen Projektionsgeometrie erstellt wird, die der gegenwärtigen Orientierung des Röntgengeräts im Raum entspricht.

## Claims

1. A method for providing a large-area X-ray image of an object supported by an object support and for providing further synthetic X-ray projections, comprising:
∘ taking a plurality of X-ray projections using an X-ray device, wherein the plurality of X-ray projections are taken during a linear movement of the object holder and/or the X-ray device;
∘ creating at least one tomosynthetic volume from the plurality of X-ray projections;
∘ creating at least one first synthetic forward projection from the at least one tomosynthetic volume, wherein the at least one synthetic forward projection produces a large-area X-ray image;
o displaying the large-area X-ray image on a suitable display device;
**characterized in that** the method further comprises:
∘ selecting and/or marking an area of interest within the large-area X-ray image;
o creating at least one second synthetic forward projection comprising the area of interest;
∘ displaying the at least one second synthetic forward projection on a suitable display device;
wherein the at least one second synthetic forward projection is created in a projection geometry corresponding to the current orientation of the X-ray device in space.

2. The method according to claim 1, **characterized in that** the linear movement of the object support and/or the X-ray device is superimposed by a further movement.

3. The method according to one of the preceding claims, **characterized in that** the movement of the object support and/or the X-ray device is detected by determining position and/or orientation information, and the reconstruction of the at least one tomosynthetic volume is performed taking this into account.

4. The method according to one of the preceding claims, **characterized in that** the at least one tomosynthetic volume is generated by means of a calibration of the recording geometry performed above.

5. The method according to one of the preceding claims, **characterized in that** the at least one second synthetic forward projection is generated in a conical beam geometry.

6. The method according to one of the preceding claims, comprising:
∘ calculating a movement of the X-ray device and/or the object positioning from the received region of interest, provided that, after the movement has been performed, a projection geometry relative to the object results which subsequently allows the production of an X-ray projection which at least approximately corresponds to the second synthetic forward projection;
∘ evaluating whether the calculated movement is feasible given the kinematic constraints of the X-ray device and/or the object support; and
∘ performing the movement if it is feasible, or not performing the movement and/or issuing a message if it is not feasible.

7. The method according to one of the preceding claims, **characterized in that** a zoom function is provided for the large-area X-ray image and/or the second synthetic X-ray projection.

8. The method according to any of the preceding claims, **characterized in that** functions for measuring lengths and/or angles on the display of the large-area X-ray image and/or the second synthetic forward projection are provided.

9. The method according to one of the preceding claims, **characterized in that** the object area represented by the second synthetic forward projection is marked at least approximately in the representation of the large-area X-ray image.

10. The method according to one of the preceding claims, **characterized in that** scene changes caused by object changes are taken into account.

11. The method according to one of the preceding claims, **characterized in that** the tomosynthetic volume is created from derived projections which contain at least one specific class of scene change and/or exclude at least one specific class of scene change.

12. The method according to any of claims 10 or 11, **characterized in that** the scene changes are caused by the temporal dynamics of a contrast agent injection.

13. The method according to claims 11 and 12, **characterized in that** at least two tomosynthetic volumes are created, one of these volumes corresponding to the state of the object without contrast agent, and another containing only the contrast agent.

14. The method according to one of the preceding claims, **characterized in that** the plurality of X-ray projections and/or projections derived therefrom are displayed within a temporal sequence.

15. The method according to one of the preceding claims, **characterized in that** the position of the currently displayed X-ray projection and/or derived projection is marked in the display of the large-area X-ray image.

16. The method according to one of the preceding claims, **characterized in that** the time rate for recording the plurality of X-ray projections and/or the pulse duration of the X-ray device is adapted to the movement, in particular to its speed and/or angular speed.

17. An X-ray device, in particular a C-arm for providing a large-area X-ray image of an object supported by an object support and for providing further synthetic X-ray projections, comprising:
- a computer program product that can be loaded into a memory device of the X-ray device;
wherein the computer program product comprises program sections for executing the following method steps:
∘ recording a plurality of X-ray projections by means of an X-ray device, wherein the plurality of X-ray projections are recorded during a linear movement of the object support and/or the X-ray device;
∘ creating at least one tomosynthetic volume from the plurality of X-ray projections;
∘ creating at least one first synthetic forward projection from the at least one tomosynthetic volume, wherein the at least one synthetic forward projection produces a large-area X-ray image;
∘ displaying the large-area X-ray image on a suitable display device;
**characterized in that** the computer program product comprises program sections for executing the following further method steps:
∘ selecting and/or marking an area of interest within the large-area X-ray image;
∘ creating at least one second synthetic forward projection comprising the area of interest;
∘ displaying the at least one second synthetic forward projection on a suitable display device;
wherein the at least one second synthetic forward projection is created in a projection geometry corresponding to the current orientation of the X-ray device in space.

## Revendications

1. Procédé pour fournir une image rayons X de grande surface d'un objet qui est supporté par un support d'objet(s) et pour fournir d'autres projections rayons X de synthèse, comprenant :
∘ la prise d'une pluralité de projections rayons X en utilisant un dispositif à rayons X, dans lequel les projections de la pluralité de projections rayons X sont prises pendant un déplacement linéaire du support d'objet(s) et/ou du dispositif à rayons X ;
∘ la création d'au moins un volume de tomosynthèse à partir de la pluralité de projections rayons X ;
∘ la création d'au moins une première projection vers l'avant de synthèse à partir de l'au moins un volume de tomosynthèse, dans lequel l'au moins une projection vers l'avant de synthèse produit une image rayons X de grande surface ; et
∘ l'affichage de l'image rayons X de grande surface sur un dispositif d'affichage approprié ;
**caractérisé en ce que** le procédé comprend en outre :
∘ la sélection et/ou le marquage d'une zone d'intérêt à l'intérieur de l'image rayons X de grande surface ;
∘ la création d'au moins une seconde projection vers l'avant de synthèse qui comprend la zone d'intérêt ; et
∘ l'affichage de l'au moins une seconde projection vers l'avant de synthèse sur un dispositif d'affichage approprié ;
dans lequel l'au moins une seconde projection vers l'avant de synthèse est créée selon une géométrie de projection qui correspond à l'orientation courante du dispositif à rayons X dans l'espace.

2. Procédé selon la revendication 1, **caractérisé en ce que** le déplacement linéaire du support d'objet(s) et/ou du dispositif à rayons X reçoit en superposition un autre déplacement.

3. Procédé selon l'une quelconque des revendications précédentes, **caractérisé en ce que** le déplacement du support d'objet(s) et/ou du dispositif à rayons X est détecté en déterminant une information de position et/ou d'orientation, et la reconstruction de l'au moins un volume de tomosynthèse est effectuée en prenant cette information en compte.

4. Procédé selon l'une quelconque des revendications précédentes, **caractérisé en ce que** l'au moins un volume de tomosynthèse est généré au moyen d'un étalonnage de la géométrie d'enregistrement selon la mention ci-avant.

5. Procédé selon l'une quelconque des revendications précédentes, **caractérisé en ce que** l'au moins une seconde projection vers l'avant de synthèse est générée selon une géométrie de faisceau conique.

6. Procédé selon l'une quelconque des revendications précédentes, comprenant :
o le calcul d'un déplacement du positionnement du dispositif à rayons X et/ou de l'objet par rapport à la région d'intérêt reçue, étant entendu que, après que le déplacement a été effectué, une géométrie de projection relative à l'objet en résulte, laquelle permet subséquemment la production d'une projection rayons X qui correspond au moins approximativement à la seconde projection vers l'avant de synthèse
o l'évaluation de si le déplacement calculé est ou non réalisable compte tenu des contraintes cinématiques du dispositif à rayons X et/ou du support d'objet(s) ; et
o la réalisation du déplacement s'il est réalisable ou la non réalisation du déplacement et/ou la délivrance d'un message s'il n'est pas réalisable.

7. Procédé selon l'une quelconque des revendications précédentes, **caractérisé en ce qu'**une fonction zoom est prévue pour l'image rayons X de grande surface et/ou la seconde projection rayons X de synthèse.

8. Procédé selon l'une quelconque des revendications précédentes, **caractérisé en ce que** des fonctions pour mesurer des longueurs et/ou des angles sur l'affichage de l'image rayons X de grande surface et/ou de la seconde projection vers l'avant de synthèse sont prévues.

9. Procédé selon l'une quelconque des revendications précédentes, **caractérisé en ce que** la zone d'objet qui est représentée par la seconde projection vers l'avant de synthèse est marquée au moins de façon approximative au niveau de la représentation de l'image rayons X de grande surface.

10. Procédé selon l'une quelconque des revendications précédentes, **caractérisé en ce que** des changements de scène qui sont générés par des changements d'objet sont pris en compte.

11. Procédé selon l'une quelconque des revendications précédentes, **caractérisé en ce que** le volume de tomosynthèse est créé à partir de projections dérivées qui contiennent au moins une classe spécifique de changement de scène et/ou qui excluent au moins une classe spécifique de changement de scène.

12. Procédé selon l'une quelconque des revendications 10 ou 11, **caractérisé en ce que** les changements de scène sont provoqués par la dynamique temporelle d'une injection d'agent de contraste.

13. Procédé selon les revendications 11 et 12, **caractérisé en ce qu'**au moins deux volumes de tomosynthèse sont créés, l'un de ces volumes correspondant à l'état de l'objet sans agent de contraste et l'autre contenant seulement l'agent de contraste.

14. Procédé selon l'une quelconque des revendications précédentes, **caractérisé en ce que** les projections de la pluralité de projections rayons X et/ou les projections qui en sont dérivées sont affichées à l'intérieur d'une séquence temporelle.

15. Procédé selon l'une quelconque des revendications précédentes, **caractérisé en ce que** la position de la projection rayons X et/ou de la projection dérivée présentement affichée(s) est marquée dans l'affichage de l'image rayons X de grande surface.

16. Procédé selon l'une quelconque des revendications précédentes, **caractérisé en ce que** la cadence pour enregistrer la pluralité de projections rayons X et/ou la durée des impulsions du dispositif à rayons X sont/est adaptée(s) par rapport au déplacement, en particulier à sa vitesse et/ou à sa vitesse angulaire.

17. Dispositif à rayons X, en particulier un bras C pour fournir une image rayons X de grande surface d'un objet qui est supporté par un support d'objet(s) et pour fournir d'autres projections rayons X de synthèse, comprenant :
- un progiciel qui peut être chargé à l'intérieur d'un dispositif de mémoire du dispositif à rayons X ;
dans lequel le progiciel comprend des sections de programme pour exécuter les étapes de procédé suivantes :
o l'enregistrement d'une pluralité de projections rayons X au moyen d'un dispositif à rayons X, dans lequel les projections de la pluralité de projections rayons X sont enregistrées pendant un déplacement linéaire du support d'objet(s) et/ou du dispositif à rayons X ;
∘ la création d'au moins un volume de tomosynthèse à partir de la pluralité de projections rayons X ;
∘ la création d'au moins une première projection vers l'avant de synthèse à partir de l'au moins un volume de tomosynthèse, dans lequel l'au moins une projection vers l'avant de synthèse produit une image rayons X de grande surface ; et
∘ l'affichage de l'image rayons X de grande surface sur un dispositif d'affichage approprié ;
**caractérisé en ce que** le progiciel comprend des sections de programme pour exécuter les autres étapes de procédé suivantes :
∘ la sélection et/ou le marquage d'une zone d'intérêt à l'intérieur de l'image rayons X de grande surface ;
∘ la création d'au moins une seconde projection vers l'avant de synthèse qui comprend la zone d'intérêt ; et
∘ l'affichage de l'au moins une seconde projection vers l'avant de synthèse sur un dispositif d'affichage approprié ;
dans lequel l'au moins une seconde projection vers l'avant de synthèse est créée selon une géométrie de projection qui correspond à l'orientation courante du dispositif à rayons X dans l'espace.
